# EUROPEAN PATENT SPECIFICATION

(11) **EP 0 864 336 B1**
(45) Date of publication and mention of the grant of the patent: **14.05.2008**
(21) Application number: 98301737.7
(22) Date of filing: 10.03.1998
(51) Int. Cl.: A61M 25/00

(54) **Catheter with valve mechanism**
Katheter mit Ventileinrichtung
Cathéter avec mécanisme à vanne

(30) Priority: 10.03.1997 US 813935
(43) Date of publication of application: 16.09.1998
(73) Proprietor: United States Surgical Corporation, Norwalk, Connecticut 06856 (US)
(72) Inventor: Person, Wayne C., Newtown, CT 06470 (US); Mastri, Dominick L., Bridgeport, CT 06606 (US); Vumback, William J., Northford, CT 06472 (US); Mozdzierz, Patrick D., Middletown, CT 06457 (US)
(74) Representative: Grey, Ian Michael

(56) References cited:
- EP-A- 0 328 332
- EP-A- 0 476 796
- EP-A- 0 583 070
- US-A- 4 936 826
- US-A- 5 147 332
- US-A- 5 224 938

## Description

This invention relates to catheters incorporating a valve mechanism to permit the ingress and egress of fluids therethrough into and out of the body of a patient.

The use of catheters in intravenous procedures and for intravenous therapies is well known in the medical community. Catheters typically are implanted into various vessels in the patients body to provide for the ingress and/or egress of fluids, such as blood and other bodily fluids, and as well for the infusion of medication or other medical solutions for both specific treatment of the patient and to facilitate other treatments and diagnoses. The use of catheters may be for short term procedures, but they are also commonly used in long term procedures wherein the catheter is implanted in the body and left in place for an extended period of time to facilitate long term treatment of the patient.

Catheters typically take the form of an elongated tube constructed of a biocompatible surgical grade material which is flexible to permit guiding or steering of the catheter through blood vessels or anatomical passages. Initially, catheters generally include an open ended tube which was positioned during the surgical procedure, and was capped at its proximal end (i.e. the end positioned outside the body) to provide a port for the infusion or withdrawal of fluids. The distal end of the catheter remained open inside the vessel within the patients body, and allowed for ready withdrawal or infusion of fluids through the catheter. These catheters were typically used in short term procedures, such as surgical procedures in which the catheter would be removed after completion of the surgical procedure. Leaving a catheter of the open-ended type in the vessel of the patient subjected the catheter to a number of potential problems, including the formation of blood clots which would obstruct the end of the catheter. Open-ended catheters are thus flushed regularly, typically with a saline and/or anticoagulant solution, to keep the distal end of the catheter open.

Catheters intended to remain in the body for a longer term have been developed and generally include a closed distal end and a valve adjacent the distal end to permit the infusion or withdrawal of fluids. Typically, these valves operate by reacting to the pressure differential within the tube as compared to the vessel (or other anatomical location) in which the catheter is placed. Generally, increasing the pressure within the catheter provides for infusion of fluids through the valve and into the vessel, while a pressure decrease in the catheter provides for withdrawal of the fluids from the site in which the catheter is placed.

A challenge associated with closed end catheters having valves adjacent their distal end is the performance of the valve based on a pressure differential. Although efforts have been made to optimise the performance of such valved catheters, e.g. by chemical weakening the area of the catheter tube adjacent to the valve or other localized treatment as disclosed in US Patent Nos. 4,549,879; 4,701,166; 4,995,863 and 5,147,332, a need remains to further optimise the fabrication and/or performance of existing valve catheters.

It is known from EP-A-0583070 A1 to provide a catheter comprising a flexible tube having a wall and defining a lumen extending therethrough and at least one valve mechanism oriented at an angle to the lontigudinal axis of the catheter which opens in response to positive or negative pressure to allow the egress and ingress of fluid through the lumen. A catheter having a lumen of constant diameter is also known from US-A-5554136.

A catheter according to the present invention is characterised in that the tube has a portion of reduced wall thickness, the at least one valve mechanism being located in said portion of reduced wall thickness in the tube, the diameter of the lumen remaining substantially constant throughout the length of the catheter, the diameter of the lumen remaining unaffected by said portion of reduced wall thickness in the tube.

Preferably, the or each valve mechanism is a slit valve.

The preferred catheter comprises an elongate flexible tube which is fabricated from a surgical grade material and has an open and closed end. The catheter tube has a wall which is defined by an inner and outer surface of the tube, where the inner surface of the tube is defined by a lumen which extends the length of the tube. In one preferred embodiment, when viewed in cross-section at two different longitudinal points, at least a portion of the tube at the more distal point has a reduced thickness with respect to the tube when viewed at a more proximal point, and at least one valve mechanism is positioned solely or entirely in this portion of reduced thickness to place the lumen in communication with the exterior of the tube.

The reduced thickness portion of the catheter tube, in a further embodiment, is the result of the lumen of the catheter tube being offset and parallel to the longitudinal axis of the tube, and in another embodiment is the result of the lumen having an oval cross-section such that the major axis of the oval defines the portions of reduced thickness in the wall of the tube. In each of these cases, the valve mechanism is provided in the portion or portions of reduced thickness, and does not extend into the areas of increased thickness so that the operation of the valve is consistent along its length.

In an alternate embodiment of the present catheter, the valve mechanism comprises at least one pair of slits which are parallel to each other but still positioned at an angle to the longitudinal axis of the catheter tube. Preferably, the slits, when formed through the tube, are cut at different angles relative to the catheter tube wall surface to facilitate the infusion or withdrawal of fluids.

In each of the embodiments, it is preferred that the valves positioned at an angle to the longitudinal axis of the catheter are located in the area of reduced thickness to increase the size of the opening for the ingress and egress of fluids.

Other features of the catheter of the present invention will become apparent from the detailed description hereafter of preferred embodiments given by way of example only with reference to the accompanying drawings, in which:
Figure 1 is a perspective view of a catheter according to a first embodiment of the invention;
Figure 2 is a top plan view of the catheter of Figure 1;
Figure 3 is a side elevation view of the catheter of Figure 1;
Figure 4 is a side cross-section view of the catheter of Figure 1 taken along lines 4-4 of Figure 2;
Figure 5 is a perspective view of a catheter according to a second embodiment;
Figure 6 is a top plan view of the catheter of Figure 5;
Figure 7 is a cross-sectional view of the catheter of Figure 5 taken along lines 11-11 of Figure 6 showing a circular lumen;
Figure 8 is a cross sectional view similar to Figure 7 showing an oval lumen;
Figure 9 is a perspective view of a catheter according to a third embodiment;
Figure 10 is a side cross-sectional view of the catheter of Figure 9 taken along lines 17-17 of Figure 9;
Figure 11 is a cross-sectional view of the catheter of Figure 9 taken along lines 18-18 of Figure 9;
Figure 12 is a perspective view of a catheter according to a fourth embodiment;
Figure 13 is a side cross-sectional view of the catheter of Figure 12 taken along lines 20-20 of Figure 13; and
Figure 14 is a cross-sectional view of the catheter of Figure 12 taken along lines 21-21 of Figure 12.

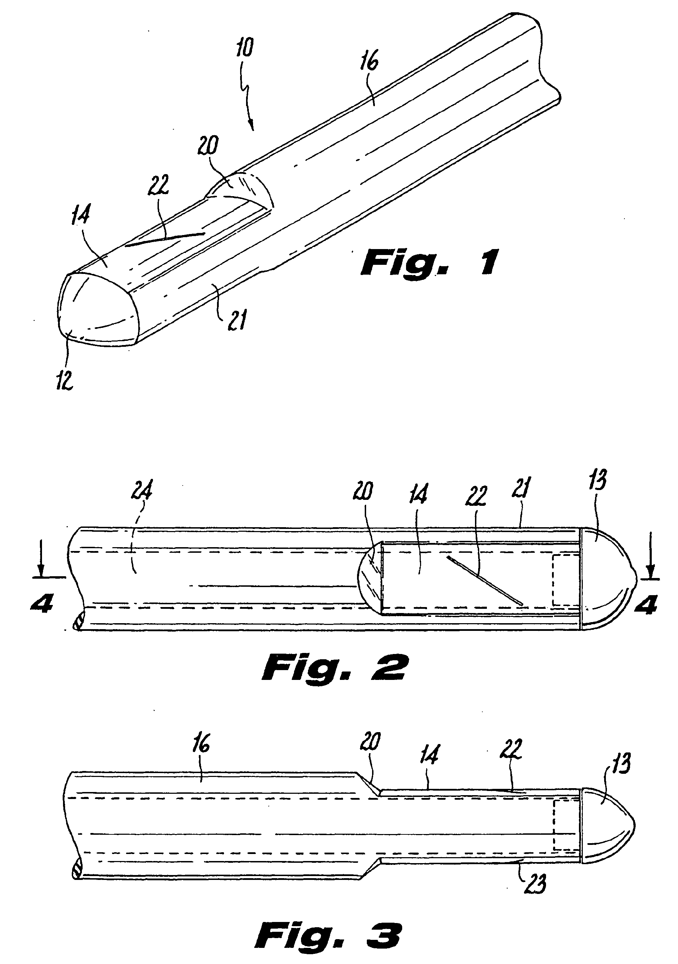

Referring now to the drawings, in which like reference numerals represent similar or identical elements throughout the several views, there is illustrated in Figure 1 a catheter 10 having a valve mechanism 22 positioned in an area of reduced thickness relative to proximal portions of catheter 10 which, in combination with its orientation to be explained hereafter, facilitates the operation of the valve mechanism to open and close for infusing or withdrawing fluids. Catheter 10 preferably is constructed of a flexible, biocompatible surgical grade material and terminates in closed distal end 12, which may take the form of an end cap 12, as seen in Figures 2-4 or may be molded as part of the catheter body 16.

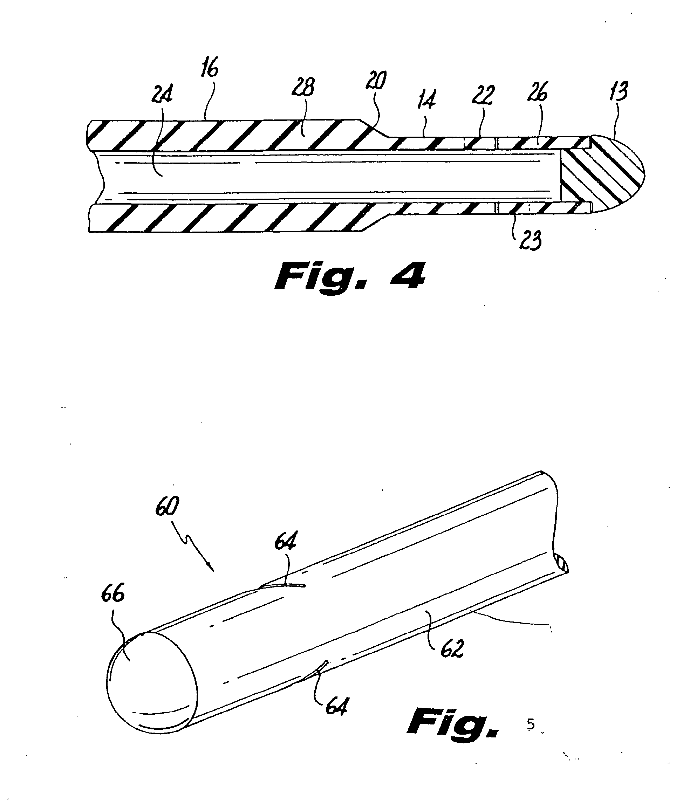

Body 16 has a first diameter which corresponds to a first thickness 28, as seen in Figure 4, of the wall of the catheter 10. A transition region 20 is provided which leads to a region 14, which is preferably substantially planar and which has a second region 26 of a reduced thickness which is less than that of the first thickness 28, as best seen in Figure 4. The reduced thickness provides added flexibility to slit valves 22,23 provided therein thereby facilitating opening and closing the valves.

Slit valves 22,23 open in response to increased or decreased pressure within lumen 24 to permit the infusion and egress of fluids into or from the catheter 10 and into the vessel in which the catheter is positioned. In the embodiment shown in Figures 3 and 4, the pair of slit valves 22,23 are cut or otherwise configured in such a manner so as to provide for infusion through one valve, i.e. valve 22, and egress through a second valve, i.e. valve 23. In other words, in this embodiment, valve 22 opens in response to increased pressure in lumen 24 and valve 23 opens in response to decreased pressure in lumen 24. Planar region 14 facilitates the opening and closing of the valves due to the reduced thickness 26 of the catheter wall, and it can be seen that, in this embodiment, the valves are positioned exclusively within the area of reduced thickness 26. In an alternate embodiment, the slit valves 22,23 are identical and the ingress and egress of fluids is through both valves.

Preferably, planar region 14 is formed in the catheter wall on diametrically opposite sides thereof. As can be seen in Figure 4, however, the reduction in wall thickness does not affect the diameter of lumen 24, which is maintained substantially constant throughout the length of catheter 10. As shown in Figure 2, the outer diameter of the catheter 10 remains constant along sides 21. Alternately, the thickness 28 of the catheter wall can be reduced circumferentially about the end of catheter 10 distally of the transition region 20, with the wall thickness being constant at this distal end of catheter 10 and the diameter of the lumen remaining constant throughout the catheter length.

Figures 1 and 2 show the valve 22 oriented at an angle to the longitudinal axis of catheter 10. Thus, valve 22 lies in a plane oriented at an angle to the longitudinal axis. Positioning the valve 22 at an angle within the reduced wall thickness results in a larger opening for the ingress and egress of fluids. When suction is applied, the reduced thickness wall will want to collapse so it will twist. Thus the slit opens into an eye-shaped opening as shown for example in Figure 1A. A preferred angular orientation of valve 22 relative to the longitudinal axis is 30 degrees, although differing angles, and particular greater angles, will provide the desired advantage.

Turning now to Figure 5, there is illustrated another embodiment of catheter 60 in which a pair of valve mechanisms 64, preferably slits, are provided in the body 62 of the catheter 60, adjacent the closed distal end 66. Each valve mechanism 64 is positioned at an angle to the longitudinal axis of the catheter 60, and preferably at a 30° angle. Optionally, valve mechanisms 64 may be provided at angles which are opposite to each other. Preferably, each such valve mechanism is positioned at an angle of approximately 30° to the longitudinal axis. Thus, in an embodiment wherein the two valve mechanisms are oriented opposite each other, the angles would be plus and minus 30 degrees relative to the longitudinal axis, respectively.

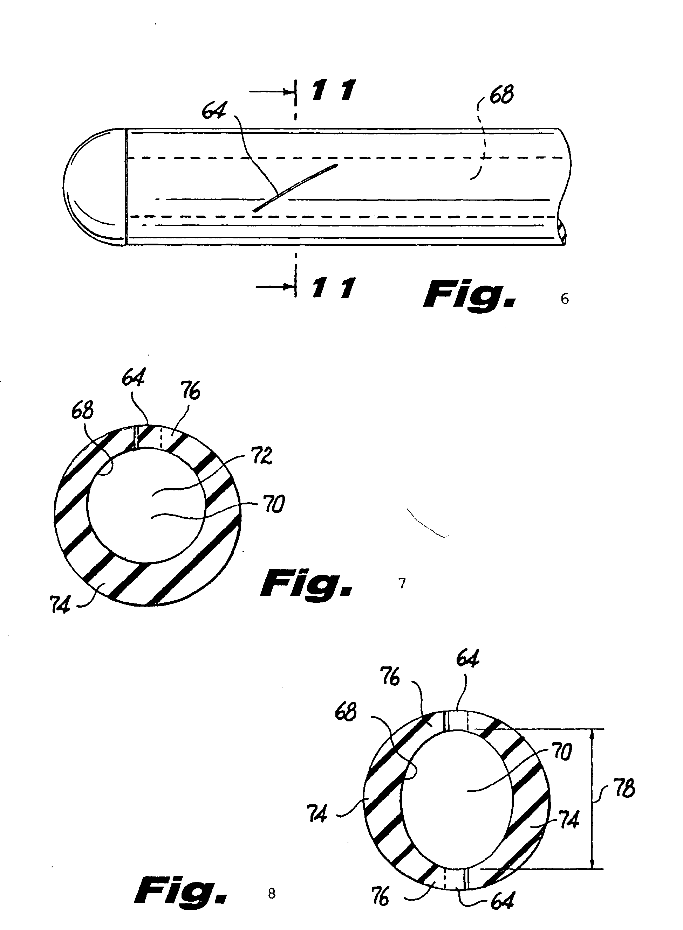

As seen in Figures 6 to 8, valve mechanism 64 is positioned exclusively or wholly within reduced thickness wall portion 76 of the catheter wall 74, and is positioned at an angle to the longitudinal axis 70. The reduced wall thickness 76 is a result, as seen in Figure 7, of extruding the catheter tubing so as to have a lumen 68 which is offset from the longitudinal axis 70 of the catheter 60. In the embodiment shown in Figure 7, the lumen 68 has a longitudinal axis 72 which is offset from the longitudinal axis 70 of the catheter 60. Wall 74 has a greater thickness than wall portion 76, and the valve mechanism 64 is positioned exclusively within the reduced thickness wall portion 76.

Figure 8 illustrates a further manner of extruding the catheter 60 in order to provide for the positioning of valve mechanisms 64 in the reduced thickness wall portion 76. In this embodiment, the lumen 68 has an oval cross-section, such that its longitudinal axis is aligned with longitudinal axis 70 of the catheter 60. The reduced thickness wall portions 76 are located at the ends of the major axis 78 of the oval shaped lumen 68, and the valve mechanisms 64 are provided at the end of the major axis 78.

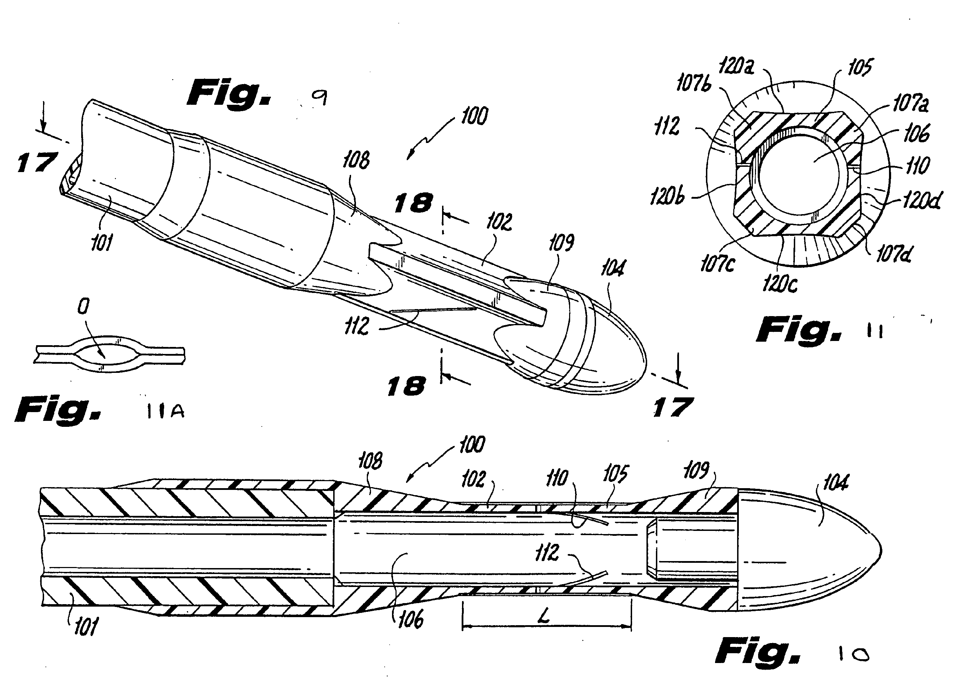

Figures 9 to 11A illustrate another alternate embodiment in which a separate valve assembly 100 is mounted e.g. by insert molding, on the tip of catheter 101 to form the catheter for insertion into the body. Valve assembly 100 includes a reduced thickness area 102 around its entire circumference. Nose 104 is configured for easier penetration, is glued to the valve assembly, and seals the distal end of the catheter and assembly 100. As shown, the reduced thickness area 102 is formed by reducing the thickness of wall 105, thereby maintaining the diameter of lumen 106 constant so as not to effect flow. Note that walls 120a-120d are slightly radiused with portions 107a-d of increased wall thickness to increase stability. The transition areas 108,109 preferably slope at an angle of about 8 to about 12 degrees to maintain stability of the catheter. A pair of diametrically valve mechanisms, preferably a pair of opposed slits 110,112 are angled with respect to the longitudinal axis (illustratively at an angle of about 24 degrees) and function as described above with respect to the embodiment of Figure 1. Thus, slit valve mechanisms 110,112 open into eye-shaped openings as shown in Figure 11A.

Length L between nose 104 and transition area 108 is selected to optimiser valve performance and in a 9mm (9 French) catheter preferably ranges from about 2.54mm to 5.08mm (0.1 to about 0.2 inches) and more preferably about 3.66mm (0.144 inches).

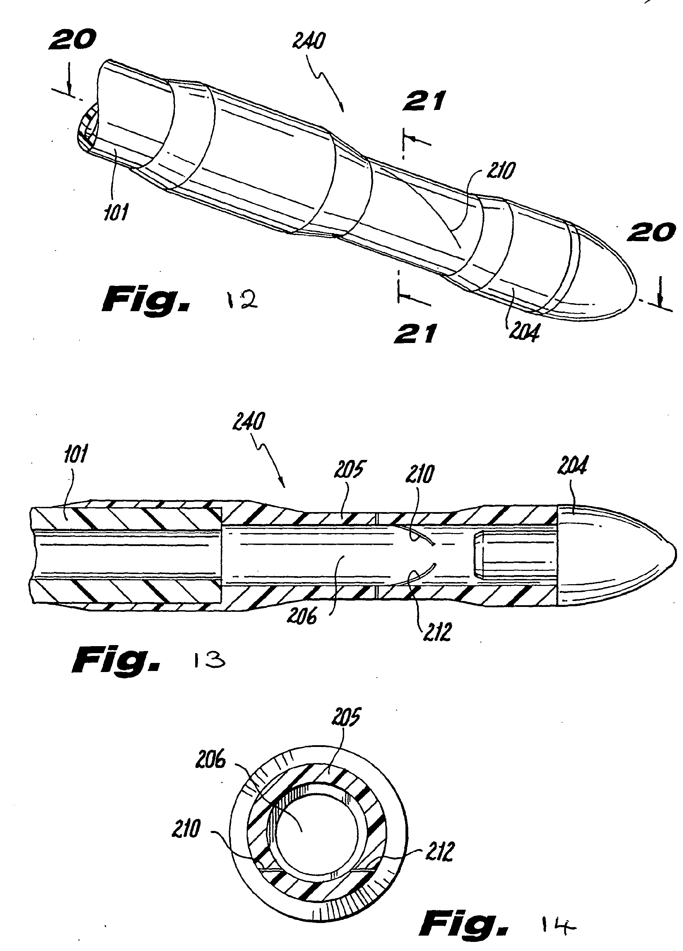

Valve assembly 240 illustrated in Figures 12 to 14 is identical to the valve assembly 100 of Figures 9 to 11A except that the reduced thickness area 202 is circular in cross section. As shown, area 202 is formed by reducing the thickness of wall 205 without effecting the internal diameter of lumen 206. Nose 204 is affixed in the same manner as nose 104. Valve mechanisms 210,212, shown as slits, are illustratively angled at about 24 degrees. As with the aforementioned embodiments, other angles are contemplated.

As noted above, the combination of an angle slit disposed on a region of reduced thickness results in a larger opening. Figure 11A illustrates by way of example the resulting eye shaped opening O which can be achieved.

## Claims

1. A catheter comprising a flexible tube having a wall and defining a lumen (24) extending therethrough and at least one valve mechanism (22) oriented at an angle to the longitudinal axis of the catheter which opens in response to positive or negative pressure to allow the egress and ingress of fluid through the lumen (24) **characterised in that** the tube has a portion (14) of reduced wall thickness, the at least one valve mechanism being located in said portion (14) of reduced wall thickness in the tube, the diameter of the lumen (24) remaining substantially constant throughout the length of the catheter (10), the diameter of the lumen (24) remaining unaffected by said portion (14) of reduced wall thickness in the tube.

2. A catheter according to claim 1, wherein the tube has a reduced diameter in said reduced thickness portion.

3. A catheter according to claim 2, **characterised in that** the reduced diameter portion (14) is circular in cross-section.

4. A catheter according to any preceding claim, **characterised by** an end cap (13) positioned on the distal end of the catheter to seal the distal end of the lumen (24).

5. A catheter according to claim 1, **characterised in that** the lumen (70) is tubular and has a circular cross-section, the longitudinal axis of the lumen being offset and parallel to the longitudinal axis of the catheter to define a portion (76) of reduced thickness in the wall (74) of the catheter.

6. A catheter according to claim 1, **characterised in that** the lumen (70) has an oval cross-section, the longitudinal axis of the lumen being aligned with the longitudinal axis of the catheter, such that the major axis of the oval defines portions (76) of reduced thickness in the wall of the catheter.

7. A catheter according to any one of the preceding claims, **characterised in that** the valve mechanism (22) is oriented at an angle of approximately 30° to the longitudinal axis of the catheters.

8. A catheter according to any one of the preceding claims, further **characterised by** a second valve mechanism (23) which is oriented at an angle of approximately 150° to the longitudinal axis of the catheter.

9. A catheter according to claim 6, further **characterised by** a second valve mechanism (64), the first valve mechanism (64) being positioned at one end of the major axis of the oval cross-section and the second valve mechanism (64) being positioned at a second end of the major axis.

10. A catheter according to claim 9, **characterised in that** the first valve mechanism is oriented at an angle of approximately 30° to the longitudinal axis of the catheter and the second valve mechanism is oriented in a plane which is at an angle of approximately 150° to the longitudinal axis of the catheter.

11. A catheter according to claim 8, **characterised in that** the first valve mechanism (64) is diametrically opposite the second valve mechanism.

12. A catheter as claimed in any one of the preceding claims, **characterised in that** the first and/or second valve mechanisms are slit valves.

## Patentansprüche

1. Katheter, der einen Schlauch umfasst und der eine Wand aufweist und ein Lumen (24), das sich dort hindurch erstreckt, und mindestens einen Ventilmechanismus (22) definiert, der in einem Winkel zu der Längsachse des Katheters ausgerichtet ist und sich in Reaktion auf Über- oder Unterdruck öffnet, um den Austritt und Eintritt von Flüssigkeit durch das Lumen (24) zu ermöglichen, **dadurch gekennzeichnet, dass** der Schlauch einen Abschnitt (14) mit verringerter Wanddicke aufweist, der mindestens eine Ventilmechanismus sich in dem Abschnitt (14) mit verringerter Wanddicke in dem Schlauch befindet, der Durchmesser des Lumens (24) über die gesamte Länge des Katheters (10) im Wesentlichen konstant bleibt, der Durchmesser des Lumens (24) von dem Abschnitt (14) mit verringerter Wanddicke in dem Schlauch unbeeinflusst bleibt.

2. Katheter nach Anspruch 1, wobei der Schlauch in dem Abschnitt mit verringerter Dicke einen verringerten Durchmesser aufweist.

3. Katheter nach Anspruch 2, **dadurch gekennzeichnet, dass** der Abschnitt mit verringertem Durchmesser (14) einen kreisförmigen Querschnitt aufweist.

4. Katheter nach einem der vorhergehenden Ansprüche, **gekennzeichnet durch** eine Abschlusskappe (13), die am distalen Ende des Katheters angeordnet ist, um das distale Ende des Lumens (24) zu verschließen.

5. Katheter nach Anspruch 1, **dadurch gekennzeichnet, dass** das Lumen (70) röhrenförmig ist und einen kreisförmigen Querschnitt aufweist, die Längsachse des Lumens versetzt ist und zu der Längsachse des Katheters parallel ist, um einen Abschnitt (76) mit verringerter Dicke in der Wand (74) des Katheters zu definieren.

6. Katheter nach Anspruch 1, **dadurch gekennzeichnet, dass** das Lumen (70) einen ovalen Querschnitt aufweist, die Längsachse des Lumens auf die Längsachse des Katheters ausgerichtet ist, so dass die Hauptachse des Ovals Abschnitte (76) mit verringerter Dicke in der Wand des Katheters definiert.

7. Katheter nach einem der vorhergehenden Ansprüche, **dadurch gekennzeichnet, dass** der Ventilmechanismus (22) in einem Winkel von ungefähr 30° zu der Längsachse des Katheters ausgerichtet ist.

8. Katheter nach einem der vorhergehenden Ansprüche, weiterhin **gekennzeichnet durch** einen zweiten Ventilmechanismus (23), der in einem Winkel von ungefähr 150° zu der Längsachse des Katheters ausgerichtet ist.

9. Katheter nach Anspruch 6, weiterhin **gekennzeichnet durch** einen zweiten Ventilmechanismus (64), wobei der erste Ventilmechanismus (64) an einem Ende der Hauptachse des ovalen Querschnitts angeordnet ist und der zweite Ventilmechanismus (64) an einem zweiten Ende der Hauptachse angeordnet ist.

10. Katheter nach Anspruch 9, **dadurch gekennzeichnet, dass** der erste Ventilmechanismus in einem Winkel von ungefähr 30° zu der Längsachse des Katheters ausgerichtet ist und der zweite Ventilmechanismus in einer Ebene ausgerichtet ist, die sich in einem Winkel von ungefähr 150° zu der Längsachse des Katheters befindet.

11. Katheter nach Anspruch 8, **dadurch gekennzeichnet, dass** der erste Ventilmechanismus (64) diametral entgegengesetzt zu dem zweiten Ventilmechanismus ist.

12. Katheter nach einem der vorhergehenden Ansprüche, **dadurch gekennzeichnet, dass** der erste und/oder der zweite Ventilmechanismus Schlitzventile sind.

## Revendications

1. Cathéter comprenant un tube flexible ayant une paroi et définissant une lumière (24) s'étendant à travers lui et au moins un mécanisme à soupape (22) orienté à un angle par rapport à l'axe longitudinal du cathéter qui s'ouvre en réponse à une pression positive ou négative pour permettre la sortie et l'entrée de fluide à travers la lumière (24), **caractérisé en ce que** le tube possède une portion (14) d'épaisseur de paroi réduite, l'au moins un mécanisme à soupape étant situé dans ladite portion (14) d'épaisseur de paroi réduite dans le tube, le diamètre de la lumière (24) demeurant essentiellement constant sur l'ensemble de la longueur du cathéter (10), le diamètre de la lumière (24) demeurant non affecté par ladite portion (14) d'épaisseur de paroi réduite dans le tube.

2. Cathéter selon la revendication 1, dans lequel le tube possède un diamètre réduit dans ladite portion d'épaisseur réduite.

3. Cathéter selon la revendication 2, **caractérisé en ce que** la portion de diamètre réduit (14) est de section transversale circulaire.

4. Cathéter selon l'une quelconque des revendications précédentes, **caractérisé par** un capuchon d'extrémité (13) positionné sur l'extrémité distale du cathéter pour sceller l'extrémité distale de la lumière (24).

5. Cathéter selon la revendication 1, **caractérisé en ce que** la lumière (70) est tubulaire et possède une section transversale circulaire, l'axe longitudinal de la lumière étant décalé et parallèle à l'axe longitudinal du cathéter pour définir une portion (76) d'épaisseur réduite dans la paroi (74) du cathéter.

6. Cathéter selon la revendication 1, **caractérisé en ce que** la lumière (70) possède une section transversale ovale, l'axe longitudinal de la lumière étant aligné avec l'axe longitudinal du cathéter de sorte que le grand axe de l'ovale définit des portions (76) d'épaisseur réduite dans la paroi du cathéter.

7. Cathéter selon l'une quelconque des revendications précédentes, **caractérisé en ce que** le mécanisme à soupape (22) est orienté à un angle d'environ 30° par rapport à l'axe longitudinal des cathéters.

8. Cathéter selon l'une quelconque des revendications précédentes, **caractérisé en outre par** un second mécanisme à soupape (23) qui est orienté à un angle d'environ 150° par rapport à l'axe longitudinal du cathéter.

9. Cathéter selon la revendication 6, **caractérisé en outre par** un second mécanisme à soupape (64), le premier mécanisme à soupape (64) étant positionné à une extrémité du grand axe de la section transversale ovale et le second mécanisme à soupape (64) étant positionné à une seconde extrémité du grand axe.

10. Cathéter selon la revendication 9, **caractérisé en ce que** le premier mécanisme à soupape est orienté à un angle d'environ 30° par rapport à l'axe longitudinal du cathéter et le second mécanisme à soupape est orienté dans un plan qui est à un angle d'environ 150° par rapport à l'axe longitudinal du cathéter.

11. Cathéter selon la revendication 8, **caractérisé en ce que** le premier mécanisme à soupape (64) est diamétralement opposé au second mécanisme à soupape.

12. Cathéter selon l'une quelconque des revendications précédentes, **caractérisé en ce que** les premier et/ou second mécanismes à soupape sont des soupapes fendues.
